(19) Europäisches Patentamt
European Patent Office
Office européen des brevets

(11) EP 2 093 288 A1

(12) **EUROPEAN PATENT APPLICATION**
published in accordance with Art. 153(4) EPC

(43) Date of publication:
**26.08.2009 Bulletin 2009/35**

(21) Application number: **07831823.5**

(22) Date of filing: **14.11.2007**

(51) Int Cl.:
***C12N 15/00*** *(2006.01)* ***C12N 1/21*** *(2006.01)*
***C12N 9/48*** *(2006.01)* ***C12N 15/09*** *(2006.01)*
***C12P 13/04*** *(2006.01)* ***C12P 19/02*** *(2006.01)*
***C12Q 1/26*** *(2006.01)* ***C12Q 1/37*** *(2006.01)*

(86) International application number:
**PCT/JP2007/072093**

(87) International publication number:
**WO 2008/059874 (22.05.2008 Gazette 2008/21)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.11.2006 JP 2006310597**

(71) Applicant: **Amano Enzyme Inc.**
**Nagoya-shi**
**Aichi 460-8630 (JP)**

(72) Inventors:
- **NISHIO, Kyouichi**
  **Kakamigahara-shi**
  **Gifu 509-0108 (JP)**
- **NAKANISHI, Yuuji**
  **Kakamigahara-shi**
  **Gifu 509-0108 (JP)**
- **TANAKA, Noriaki**
  **Kakamigahara-shi**
  **Gifu 509-0108 (JP)**

(74) Representative: **Bublak, Wolfgang**
**Bardehle, Pagenberg, Dost, Altenburg, Geissler**
**Galileiplatz 1**
**81679 München (DE)**

# (54) NOVEL DIPEPTIDE-DIGESTING ENZYME, METHOD OF PRODUCING THE SAME, METHOD OF ASSAYING GLYCATED PROTEIN BY USING THE DIPEPTIDE-DIGESTING ENZYME AND REAGENT COMPOSITION TO BE USED THEREIN

(57) Provided are a dipeptide-degrading enzyme which acts on a dipeptide to produce amino acids; a novel measurement method for quickly, simply, and accurately measuring a glycosylated protein, a glycosylated peptide, or a glycosylated dipeptide, including allowing the above enzyme to act on a glycosylated peptide to produce a glycosylated amino acid and an amino acid, and quantifying the amino acids; a dipeptide-degrading enzyme which acts on a dipeptide to produce amino acids, the dipeptide being a glycosylated dipeptide, and the amino acids including a glycosylated amino acid and an amino acid; a method for producing a glycosylated amino acid and an amino acid, including allowing a protease and a dipeptide-degrading enzyme to act on a specimen containing a glycosylated protein or a glycosylated peptide; a method for measuring a glycosylated protein, a glycosylated peptide, or a glycosylated dipeptide, including allowing the amino acid produced by the above method to be degraded by a substance which degrades the amino acid, and quantifying the amino acid.

Fig.7

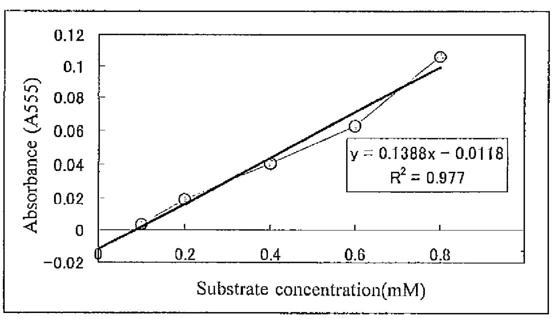


EP 2 093 288 A1

## Description

### Technical Field

The present invention relates to a novel enzyme which acts on a dipeptide, and specifically to a dipeptide-dograding enzyme which degrades a dipeptide into amino acids, a method for producing the same, a method for producing a glycosylated amino acid and an amino acid through the degradation of a glycosylated dipeptide, a method for measuring a glycosylated protein, a glycosylated peptide, and a glycosylated dipeptide, and a reagent composition to be used for the measurement.

### Background Art

In recent years, the number of diabetics is exploding. In the diagnosis and control of diabetes, the measurement of blood glucose control markers, such as glycosylated hemoglobins (glycohemoglobins; GHbs), glycoalbumins, fructosamines, and 1,5-anhydroglucitol, is very important. Therefore, there are increasing demands for the measurement of these blood glucose control markers. In particular, it is known that the concentration of glycosylated hemoglobins in the blood reflects the average blood glucose level over the past one to two months. Recent studies have proofed that the risk rates of developments of complications and diabetes are significantly decreased by keeping the blood glycosylated hemoglobin level at 7% or lower. Now the blood glycosylated hemoglobin level is an indispensable indicator in clinical fields.
Glycosylated hemoglobins are Amadori compounds formed by glycosylation of hemoglobins by Maillard reaction, and are known to be formed by ε glycosylation of N-terminal valine in the α and β chains and intramolecular lysine of hemoglobin polypeptides. Among glycosylated hemoglobins, those formed by glycosylation of N-terminal valine in the β chain of a hemoglobin are called hemoglobin A1c (HbA1c). Since HbA1c is a stable Amadori compound, it is used for the measurement of glycosylated hemoglobins. HbA1c does not include unstable hemoglobins, which are Schiff base intermediate before Amadori rearrangement. The amadori compound includes a ketoamine structure expressed by "-(CO)-CHR-NH- (wherein R represents a hydrogen atom or a hydroxy group)" obtained by Maillard reaction between an amino group-containing compound such as a peptide or protein and an aldehyde group-containing compound such as glucose.

Known methods for measuring a glycosylated hemoglobin include chromatographic methods such as ion exchange chromatography and affinity chromatography, electrophoretic methods, immunization methods, methods using reducibility of glycosylated protein in an alkaline environment, methods using thiobarbituric acid, and enzymatic methods. The methods using high performance liquid chromatography, affinity chromatography, or electrophoresis are not suitable for clinical tests using many specimens, because they require expensive special apparatuses and take much time for the measurement. The immunization methods are recently prevailing because their analysis procedures are relatively simple and the measurement takes only a short time. However, the accuracy of these methods is not satisfactory. The methods using reducibility or thiobarbituric acid are susceptible to the coexisting substances in the specimen, and are thus have problems with specificity. In comparison with these methods, the enzymatic methods are advantageous in that they do not require special apparatus, take only a short time for the measurement, provide great accuracy, simplicity, and cost efficiency. A known enzymatic method is a method for measuring a glycosylated amino acid using a fructosyl amino acid oxidase (hereinafter may be abbreviated as "FAO"), which is an enzyme acts on glycosylated amino acids (see Patent Documents 1 to 10). FAO strongly acts on glycosylated amino acids, but does not act on glycosylated proteins, glycosylated peptides, or glycosylated dipeptides (see Non-Patent Document 1). Therefore, it is impossible to measure amino acids derived from glycosylated proteins, glycosylated peptides, or glycosylated dipeptides with FAO alone. On that account, such a glycosylated protein or glycosylated peptide must be degraded by a chemical or enzymatic method into glycosylated amino acids to be released before the treatment with FAO. Specifically, the measurement of a glycosylated hemoglobin includes steps of degrading the glycosylated hemoglobin with any protease, liberating the resultant glycosylated peptide, glycosylated amino acid, and amino acid (degradation by protease), allowing FAO to act on the glycosylated amino acid, and then measuring the generated hydrogen peroxide using the color development by a peroxidase (color development by FAO).
Degradation by protease:

Glycosylated hemoglobin → glycosylated peptide → glycosylated dipeptide → glycosylated amino acid + amino acid

Color development by FAO:

glycosylated amino acid → glucosone + Val + $H_2O_2$

Patent Document 1: Japanese Examined Patent Application Publication No. 5-3 3997
Patent Document 2: Japanese Examined Patent Application Publication No. 6-65300
Patent Document 3: Japanese Unexamined Patent Application Publication No. 2-195900
Patent Document 4: Japanese Unexamined Patent Application Publication No. 3-155780
Patent Document 5: Japanese Unexamined Patent Application Publication No. 5-192193
Patent Document 6: Japanese Unexamined Patent Application Publication No. 6-46846
Patent Document 7: Japanese Unexamined Patent Application Publication No. 7-289253
Patent Document 8: Japanese Unexamined Patent Application Publication No. 8-154672
Patent Document 9: Japanese Unexamined Patent Application Publication No. 8-336386
Patent Document 10: Japanese Unexamined Patent Application Publication No. 2005-110657
Non-Patent Document 1: Biotechnology Letters 27: 963 (2005)

Disclosure of Invention

Problems to be Solved by the Invention

However, it is known that proteases are less effective for shorter peptide chains. Proteases cannot degrade glycosylated Val-His (Fru-Val-His, fructosyl valyl histidine), glycosylated Val-His-Leu-Thr-Pro-Glu (Fru-VHLTPE), and the like, which are glycosylated hemoglobin β chain dipeptides, into glycosylated amino acids, or slightly degrade them to produce a very little amount of degradation products. In these cases, a large amount of a protease must be reacted for a long time (see Non-Patent Document 1). A protease acts on a glycosylated hemoglobin to produce a glycosylated peptide or a glycosylated dipeptide, but can hardly degrade them into glycosylated Val, which is a glycosylated amino acid used as the substrate of FAO. Therefore, the above-described method using a protease and FAO has a problem with sensitivity.

The present invention has been accomplished in view of the above-described circumstances, and is intended to provide a dipeptide-degrading enzyme which degrades a dipeptide into amino acids, and a novel method for quickly, simply, and accurately measuring a glycosylated protein, a glycosylated peptide, or a glycosylated dipeptide, including allowing the enzyme to act on a glycosylated peptide to produce a glycosylated amino acid and an amino acid, and quantifying the amino acids. The present invention is also intended to improve the sensitivity of the measurement of a glycosylated hemoglobin using the dipeptide-degrading enzyme in the enzymatic method wherein a glycosylated hemoglobin is treated with a protease and FAO.

Means for Solving the Problem

In order to solve the above-described problems, the inventors searched a dipeptide-degrading enzyme capable of degrading glycosylated Val-His into glycosylated Val and histidine. As a result of screening on various natural microorganisms and enzymes contained in commercially available exogenous enzymes, a bacterium producing a dipeptide-degrading enzyme was found. The present invention has thus been accomplished.
More specifically, the present invention relates to a dipeptide-degrading enzyme which acts on a dipeptide to produce amino acids. In the invention, when the dipeptide is a glycosylated dipeptide, a glycosylated amino acid and an amino acid are produced. When the glycosylated dipeptide is glycosylated Val-His, which is a glycosylated hemoglobin β chain dipeptide, glycosylated Val and histidine are produced. The glycosylated dipeptide and glycosylated amino acid referred herein are glycosylated products of a dipeptide and an amino acid, respectively. The glycosylated Val-His is fructosyl valyl histidine (also referred to as "Fru-Val-His"). Glycosylated Val is fructosyl valine (also referred to as "Fru-Val").

An aspect of the present invention is a dipeptide-degrading enzyme having the following physicochemical properties.

(1) Action and substrate: The dipeptide-degrading enzyme acting on a dipeptide to produce amino acids, and acting on a glycosylated dipeptide to produce a glycosylated amino acid and an amino acid.
(2)Optimum pH: The optimum pH of the dipeptide-degrading enzyme is 6.2, and the activity of the enzyme in the pH range of 6.0 to 7.0 is 80% or more of the activity at pH6.2.
(3) pH stability: The dipeptide-degrading enzyme is stable at pH 5.0 or higher.
(4) Optimum temperature: The optimum temperature of the dipeptide-degrading enzyme is at 45°C, and the enzymatic reaction adequately proceeds in the temperature range of 35 to 45°C.
(5) Heat stability: The dipeptide-degrading enzyme stably keeps its activity at 50°C or lower.
(6) Molecular weight: The molecular weight of the dipeptide-degrading enzyme is about 45k Da (SDS-PAGE).

(7) Activator and inhibitor: The dipeptide-degrading enzyme is activated by $Mg^{2+}$, $Co^{2+}$, or $Zn^{2+}$ ions, and inhibited by EDTA or α,α'-dipyridyl.

The present invention relates to a dipeptide-degrading enzyme derived from Sphingopyxis chilensis No. 0522 (NITE BP-250) or a mutant of the strain.

The present invention also relates to a protein having an amino acid sequence indicated by SEQ ID No. 2 in the sequence listing.

The present invention also relates to a gene coding for the protein having the amino acid sequence indicated by SEQ ID No. 2 in the sequence listing. The present invention also relates to a DNA having the base sequence indicated by SEQ ID No. 1 in the sequence listing.

The present invention also relates to a method for producing a dipeptide-degrading enzyme, including culturing a microorganism belonging to genus Sphingopyxis, allowing the dipeptide-degrading enzyme to be produced, and collecting the dipeptide-degrading enzyme.

The present invention also relates to a method for producing a glycosylated amino acid and an amino acid, including allowing the dipeptide-degrading enzyme or the protein to act on a specimen containing a glycosylated dipeptide. The present invention also relates to a method for producing a glycosylated amino acid and an amino acid, including allowing a protease and the dipeptide-degrading enzyme or the protein to act on a specimen containing a glycosylated protein or a glycosylated peptide.

The present invention also relates to a method for measuring a glycosylated protein, a glycosylated peptide, or a glycosylated dipeptide, including allowing the amino acids produced by the above producing method to be degraded by a substance that degrades the amino acids, and quantifying the amino acids. The present invention also relates to a method for measuring a glycosylated protein, a glycosylated peptide, or a glycosylated dipeptide, including using an oxidase to quantify the thus-produced glycosylated amino acid.

The present invention also relates to a reagent composition to be used for carrying out the measurement method.

Advantageous Effect of the Invention

The dipeptide-degrading enzyme of the present invention allows quick, simple, and accurate measurement of a glycosylated protein such as a glycosylated hemoglobin, a glycosylated peptide, or a glycosylated dipeptide, and thus is suitable for the diagnosis and control of symptoms such as diabetes.

In addition, the dipeptide-degrading enzyme of the present invention improves the sensitivity of the measurement of a glycosylated hemoglobin in an enzymatic method wherein a glycosylated hemoglobin is treated with a protease and FAO, and thus is suitable for the diagnosis and control of diabetes symptoms for which conventional enzymatic methods are used.

Brief Description of the Drawings

Fig. 1 is a graph showing the optimum pH for the dipeptide-degrading enzyme.
Fig. 2 is a graph showing the pH stability of the dipeptide-degrading enzyme.
Fig. 3 is a graph showing the optimum temperature for the dipeptide-degrading enzyme.
Fig. 4 is a graph showing the thermal stability of the dipeptide-degrading enzyme.
Fig. 5 shows a migration pattern of the dipeptide-degrading enzyme analyzed by SDS-PAGE.
Fig. 6 shows a restriction enzyme map of the dipeptide-degrading enzyme gene in the chromosomal DNA of Sphingopyxis chilensis No. 0522.
Fig. 7 shows the result of the measurement of a glycosylated dipeptide (Fru-Val-His).
Fig. 8 shows response curves of Fru-Val-His and Fru-Val-Leu.

Best Mode for Carrying Out the Invention

The dipeptide-degrading enzyme of the present invention catalyzes the acting on a dipeptide to produce amino acids. When the dipeptide is a glycosylated dipeptide, the enzyme catalyzes the degradation of the glycosylated dipeptide into a glycosylated amino acid and an amino acid. The substrate specificity is characteristic of the enzyme, and cannot be found in known proteases. Accordingly, the dipeptide-degrading enzyme is useful in a novel method for measuring a glycosylated protein or a glycosylated peptide based on the quantification of amino acids produced from a glycosylated peptide, which is formed by fragmentation of a glycosylated protein or a glycosylated peptide by a protease. The dipeptide-degrading enzyme acts on a glycosylated peptide, which cannot be measured by FAO, thus feeding the substrate for FAO. As a result of this, the measurement sensitivity of the enzymatic method using FAO is improved. Accordingly, the combination of the dipeptide-degrading enzyme with a protease or an amino acid oxidase, etc., or with a protease and FAO is markedly useful and preferable for the measurement of a glycosylated hemoglobin. Details of the case where a dipeptide is the glycosylated dipeptide will be described below. When the dipeptide to be degraded by the dipeptide-degrading enzyme is an glycosylated dipeptide, the dipeptide-degrading enzyme may be referred to as a glycosylated dipeptide-degrading enzyme.

The dipeptide-degrading enzyme occurs widely in nature, and examples of the source include microorganisms. Enzyme proteins derived from Sphingopyxis are preferred, and an enzyme protein derived from Sphingopyxis chilensis No. 0522 is particularly preferred.

Sphingopyxis chilensis NO. 0522 is a novel microorganism isolated from soil by the inventors. It is internationally deposited with the following international depositary under "the Budapest Treaty on the International Recognition of the Deposit of Microorganisms for the purposes of Patent Procedure", and is readily available therefrom.

International depositary: NITE Patent Microorganisms Depositary (NPMD)

Address: 2-5-8 Kazusakamatari, Kisarazu, Chiba, Japan 292-0818

http://www.nbrc.nite.go.jp/npmd/

Date of deposit: July 19, 2006

Deposit number: NITE BP-250

In order to produce the dipeptide-degrading enzyme, firstly, a microorganism capable of producing the dipeptide-degrading enzyme is cultured (culturing step). The microorganism may be, for example, Sphingopyxis chilensis NO. 0522 described above. The culture method and culture conditions are not particularly limited as long as the intended enzyme is produced. Namely, the culture method and culture conditions that are suitable for the microorganism to be used may be appropriately established so as to allow the microorganism to sufficiently produce the enzyme. Examples of the culture conditions will be described below.

The culture medium is not particularly limited as long as it sufficiently grows the microorganism. The culture fluid appropriately contains a carbon source, a nitrogen source, an inorganic substance, and other nutrients, and may be a synthetic or natural medium. Examples of the carbon source include glucose, fructose, xylose, and glycerol. Examples of the nitrogen source to be preferably used include nitrogen-containing organic substances such as peptone, casein digests, and yeast extracts. Examples of the inorganic substance include salts of sodium, potassium, calcium, manganese, magnesium, iron, and cobalt. In order to promote the growth of the microorganism, the culture medium may contain a vitamin or an amino acid. The pH of the culture medium is adjusted to, for example, about 3 to 8, preferably about 6.5 to 7.0, the culture temperature is usually from 10 to 50°C, preferably from about 28 to 30°C, and the culture period is usually from 20 to 48 hours, preferably from 32 to 40 hours, under aerobic conditions. The culture method may be shaking culture, deep culture using a jar fermenter under aerobic conditions, anaerobic culture, or solid culture.

After the culturing step, the dipeptide-degrading enzyme is collected from the bacterial cells during culture (collection step). Since the dipeptide-degrading enzyme accumulates in the bacterial cells, the enzyme may be collected through an appropriate combination of common processes, such as fragmentation of the bacterial cells by autolysis, freezing, ultrasonic fragmentation, or pressurization, followed by ammonium sulfate precipitation, precipitation using an organic solvent such as ethanol, or any chromatography such as ion exchange chromatography, hydrophobic chromatography, gel filtration chromatography, or affinity chromatography. More specifically, the culture fluid after culture is centrifuged to collect bacterial cells, the bacterial cells are washed, suspended in a 0.1 M 3-Morpholinopropanesulfonic acid (MOPS) buffer (pH 7.0), and then fragmented by ultrasonic fragmentation. Subsequently, the solution containing the fragmented bacterial cells is centrifuged to obtain a supernatant (cell-free extract), and the supernatant is subjected to ammonium sulfate precipitation, anion exchange chromatography, and gel filtration chromatography, thereby obtaining a purified enzyme protein of the dipeptide-degrading enzyme.

Using the purified dipeptide-degrading enzyme, the amino acid sequence and gene sequence of the enzyme are determined by a known method, and a gene coding for the dipeptide-degrading enzyme is prepared. Known techniques for the genetic engineering and cell culture may be used. For example, handling of plasmids, DNA, various enzymes, coliform bacteria, and cultured cells may follow the methods described in "Current Protocols in Molecular Biology, F. Ausubel et al., (1994), John Wiley & Sons, Inc.", and "Culture of Animal Cells; A Manual of Basic Technique, R. Freshney, second edition (1987), Wiley-Liss". For example, a purified enzyme is degraded by a common procedure such as Edman degradation, thereby determining the amino acid sequence at the amino terminals. Further, the degraded enzyme is digested with a protease such as lysyl endopeptidase, and the peptide fragment is analyzed by HPLC to determine the internal amino acid sequence of the protein. On the basis of the amino acid sequence thus determined, a synthetic DNA is prepared. Using the DNA as the primer or probe, a gene coding for the dipeptide-degrading enzyme is cloned, thereby obtaining an intended gene. In the present invention, the amino acid sequence of the dipeptide-degrading enzyme is not particularly limited as long as it has the enzymatic activity. Examples of the amino acid sequence include the above-described one and those obtained by deletion, substitution, or addition of one or several amino acid residues in the amino acid sequence. In the present invention, the gene coding for the dipeptide-degrading enzyme may be consisted of a DNA coding for a protein having the enzymatic activity, and includes the above-described one, and those obtained by deletion, substitution, or addition of one or several bases of the gene, and code for a protein having the enzymatic activity. Also included are genes consisted of a DNA having a predetermined base sequence, those obtained by deletion, substitution, or addition of one or several bases of the gene, and code for a protein having the enzymatic activity, and those hybridized with a DNA of the gene under stringent conditions, and code for a protein having enzymatic activity. The stringent conditions include (a) hybridization at 42°C for 16 hours using DIG EASY Hyb (Roche Diagnostics K.K.); (b) washing twice for 5 minutes each in 2 × SSC containing 0.1% SDS at room temperature; and (c) washing for 5 minutes at 68°C with 0.1 × SSC containing 0.1% SDS.

The entire base sequence of the cloned gene coding for the dipeptide-degrading enzyme is determined using a DNA sequencer, and the primary sequence of the amino acid of the dipeptide-degrading enzyme is deduced on the basis of the gene information.

In order to obtain a gene which codes for an amino acid sequence having the activity of the dipeptide-degrading enzyme obtained by deletion, substitution, or addition of one or several amino acid residues in the amino acid sequence determined above, various known methods may be used. Examples of the known method include site-specific mutagenesis wherein a point or deletion mutant of the gene is created, and oligonucleotide mutagenesis wherein the gene is selectively cleaved, a selected nucleotide is deleted, substituted, or added, and then the gene is restored.

A DNA fragment containing the above-described gene coding for the dipeptide-degrading enzyme is incorporated into a recombinant vector, and a microorganism is transformed or transduced by the recombinant vector into a transformant or transductant producing the dipeptide-degrading enzyme. The dipeptide-degrading enzyme is produced by the transformant or transductant. The host used for the transformation or transduction is not particularly limited, and may be Escherichia coli, a strain belonging to genus Escherichia, or a strain belonging to genus Pseudomonas. A liquid culture method is suitable for culturing the transfonnant and the like.

The culture medium for culturing the transformant is not particularly limited as long as it appropriately contains a carbon source, a nitrogen source, inorganic substances, and other nutrients, and may be selected from any synthetic or natural media. Examples of the carbon source include glucose, fructose, xylose, and glycerol, and examples of the nitrogen source include nitrogen-containing organic substances such as peptone, casein digests, and yeast extracts. Examples of the inorganic substance include salts of sodium, potassium, calcium, manganese, magnesium, iron, and cobalt. The culture conditions are not particularly limited as long as the transformant or the like sufficiently grows to produce the dipeptide-degrading enzyme. The pH of the culture medium is preferably from 7.0 to 9.0, the culture temperature is usually from 25 to 42°C, preferably from 30 to 37°C, and the culture period is usually from 8 to 48 hours, preferably from 16 to 30 hours.

With the use of an appropriate combination of known techniques, the dipeptide-degrading enzyme is collected from a cultured transformant or the like, and purified. For example, after culturing a transformant or the like, bacterial cells are collected from the culture fluid by filtration, centrifugation, etc., and then the bacterial cells are fragmented. The fragmentation of the bacterial cells may be carried out by a mechanical means, autolysis using a solvent, freezing, ultrasonic fragmentation, pressurization, cell wall digestion by a cell wall digesting enzyme such as lysozyme, or a method using a surfactant such as TRITON X-100. The dipeptide-degrading enzyme is purified from the fragments of bacterial cells through an appropriate combination of processes, for example, ammonium sulfate precipitation, precipitation using an organic solvent such as ethanol, various types of chromatography such as ion exchange chromatography, hydrophobic chromatography, gel filtration chromatography, and affinity chromatography, and electrophoresis.

The dipeptide-degrading enzyme is usable for measurement of a glycosylated protein, a glycosylated peptide, or a glycosylated dipeptide. The specimen to be measured is not particularly limited as long as it is a glycosylated protein, a glycosylated peptide, or a glycosylated dipeptide. Examples of preferred specimens include urine and blood components such as whole blood, blood cells, erythrocytes, hemolysins, blood serums, and plasmas. The method for measuring a glycosylated protein, a glycosylated peptide, or a glycosylated dipeptide uses the following reagents. [0031] Reagent A: a reagent containing a substance having protease activity for degrading a glycosylated protein or glycosylated peptide into a glycosylated dipeptide. The dipeptide-degrading enzyme acts exclusively on a dipeptide. Therefore, when the glycosylated protein or glycosylated peptide is to be measured, the test substance is degraded into a glycosylated dipeptide in advance. The test substance may be degraded by a protease. The protease may be an endoprotease which degrades a protein from the inside of the polypeptide chains, or an exoprotease which degrades a protein from the outside of the polypeptide chains. Examples of the endoprotease include trypsin, $\alpha$-chymotrypsin, subtilisin, proteinase K, papain, cathepsin B, pepsin, thermolysin, protease, protease XVII, protease XXI, lysyl endopeptidase, and examples of the exoprotease include aminopeptidase and carboxypeptidase. The enzyme is not particularly limited as long as it has protease activity. The protease activity has only to degrade a glycosylated protein or a glycosylated peptide, and is from 0.1 to 10000 u/mL, preferably from 1 to 1000 u/mL, and more preferably from 10 to 500 u/mL. The pH of the reagent A may be adjusted in consideration of the stability and reaction efficiency of the enzyme, and is usually from 4.0 to 8.0, preferably from 5.0 to 7.0, and more preferably from 5.5 to 6.5.

Reagent B: a reagent containing a substance which eliminates amino acids produced by the degradation of a glycosylated protein or a glycosylated peptide by a protease. The reagent B may use any system as long as it eliminates amino acids. For example, the reagent B contains 0.01 to 1000 u/mL, preferably 0.1 to 500 u/mL, more preferably 1 to 100 u/mL of an amino acid oxidase; 0.01 to 1000 u/mL, preferably 0.1 to 100 u/mL, more preferably 1 to 10 u/mL of a catalase; and 4-aminoantipyrine (4-AA). The pH of the reagent B may be adjusted in consideration of the stability and reaction efficiency of the enzyme, and is usually from 5.5 to 9.0, preferably from 6.5 to 8.5, and more preferably from 7.0 to 8.0. The substance eliminating amino acids may be the oxidase described above, or a dehydrogenase.

When hydrogen peroxide is generated as a result of the elimination of amino acids, the reagent B may contain a catalase thereby degrading the hydrogen peroxide as described above.

Reagent C: a reagent containing the dipeptide-degrading enzyme and substances for degrading and quantifying an amino acid or a glycosylated amino acid produced by degradation of a glycosylated dipeptide by the dipeptide-degrading enzyme. When dehydrogenase is used as the enzyme degrading the amino acid, the change of the amount of the coenzyme may be directly or indirectly measured. More specifically, when nicotinamide adenine dinucleotide (NAD) is used as the coenzyme, the maximum absorption wavelength (340 nm) of the reduced NAD is directly quantified with a chromometer, or reduced NAD is indirectly or directly quantified using an electron carrier such as diaphorase or PMS and any reduced coloring reagent such as a tetrazolium salt. When an oxidase is used as the enzyme acting on the amino acids, the oxygen consumption or the reaction product is measured. For example, when an amino acid oxidase is used, hydrogen peroxide, ammonia, or 2-oxoacid formed by the reaction may be directly or indirectly measured by a known method. In addition, when FAO is used as the enzyme for degrading the glycosylated amino acid, the hydrogen peroxide produced may be measured. Examples of the method for measuring hydrogen peroxide include the followings.

1. Color development method: a method using a Trinder's reagent which develops a color by the oxidation/condensation reaction between a coupler such as 4-aminoantipyrine (4-AA) or 3-methyl-2-benzothiazolinone hydrazone (MBTH) and a chromogen such as phenol in the presence of a peroxidase (POD), or a leuco reagent which directly causes oxidative coloration.
2. Fluorescence method: a method using a compound which shows fluoresce upon oxidation. Examples of the compound include homovanillic acid, 4-hydroxyphenyl acetic acid, tyramine, para cresol, and diacetyl fluorescin derivatives.
3. Chemiluminescence method: a method using luminol, lucigenin, isoluminol, pyrogallol, etc., as a catalyst.

The reagent C is preferably composed of 0.1 to 1000 u/mL, preferably 0.5 to 100 u/mL, more preferably 1 to 10 u/mL of the dipeptide-degrading enzyme; 0.1 to 1000 u/mL, preferably 1 to 100 u/mL of a L-amino acid oxidase which oxidizes free amino acids; 0.1 to 1000 u/mL, preferably 1 to 100 u/mL, more preferably 5 to 50 u/mL of a peroxidase; and 0.05 to 0.5% of N-ethyl-N-(2-hydroxy-3-sulfopropyl)-3-methylaniline sodium salt (TOOS) and sodium azide ($NaN_3$). The sodium azide contained at the above-described concentration deactivates the catalase contained in the reagent B. The pH of the reagent C may be adjusted in consideration of, for example, the stability and reaction efficiency of the enzyme, and is usually from 5.0 to 9.0, preferably from 6.0 to 8.0, and more preferably from 6.5 to 7.5. The reagents A, B, and C may contain one or more of a surfactant, a nonionic surface-active agent, and an amphoteric surfactant at a concentration of 0.0005% to 10%.

Regarding the components of the reagent C, the dipeptide-degrading enzyme may be contained in different reagent from the substances for degrading and quantifying the amino acid or glycosylated amino acid produced through the degradation of the glycosylated dipeptide by the dipeptide-degrading enzyme.

(1) Measurement of glycosylated protein or glycosylated peptide

An embodiment of the measurement of a glycosylated protein or a glycosylated peptide using the reagents A, B, and C will be described below.

First reaction: The reagent A is added to a specimen containing 0.001 to 1.0 mL of a glycosylated protein or glycosylated peptide, thereby degrading the glycosylated protein or glycosylated peptide into a glycosylated dipeptide (formula (1)). The reaction temperature is not particularly limited as long as the glycosylated protein or glycosylated peptide contained in the specimen is degraded by a protease, and is usually from 20 to 45°C, and preferably from 30 to 40°C. The reaction time is usually from 0.1 to 60 minutes, and preferably from 1 to 10 minutes.

Second reaction: The reagent B is added to the reaction solution obtained by the first reaction, thereby eliminating the free amino acids produced by the first reaction (formula (2)).

Third reaction: The reagent C is added to the specimen obtained by the second reaction, allowing the dipeptide-degrading enzyme to liberate a glycosylated amino acid and an amino acid from the glycosylated dipeptide. Subsequently, the free amino acids are oxidized by L-amino acid oxidase to generate a hydrogen peroxide, and 4-AA and TOOS are oxidized and condensed by the hydrogen peroxide and peroxidase, thus quantitatively forming a quinone pigment having an absorbance at 555 mn. The change in the absorbance of the quinone pigment at 555 nm is measured and qualified (formula (3)).

Formula (1): glycosylated protein (glycosylated peptide) → glycosylated dipeptide + free amino acid

Formula (2): free amino acid + $O_2$ + $H_2O$ → α-keto acid + ammonia + hydrogen peroxide

Formula (3): glycosylated Val-His → glycosylated Val + L-His L-His + $O_2$ + $H_2O$ → α-keto acid + ammonia + hydrogen peroxide 2Hydrogen peroxide + 4-AA + TOOS → quinone pigment + $4H_2O$

(2) Measurement of glycosylated dipeptide

An embodiment of the measurement of a glycosylated dipeptide using the reagents B and C will be described below.

First reaction: The reagent B is added to 0.001-1.0 mL of a specimen solution containing a glycosylated dipeptide, and allowing the reagent B to react (formula (2)). The reaction temperature is not particularly limited as long as the free amino acids contained in the specimen solution are eliminated, and is usually from 20 to 45°C, and preferably from 30 to 40°C. The reaction time is usually from 0.1 to 60 minutes, and preferably from 1 to 10 minutes. When no amino acid is contained in the specimen, the treatment with the reagent B may be omitted.

Second reaction: The reagent C is added to the reaction solution obtained by the first reaction, allowing the dipeptide-degrading enzyme to liberate a glycosylated amino acid and an amino acid from the glycosylated dipeptide. Subsequently, the free amino acids are oxidized by L-amino acid oxidase to generate a hydrogen peroxide, and 4-AA and TOOS are oxidized and condensed by the hydrogen peroxide and peroxidase, thus quantitatively forming a quinone pigment having an absorbance at 555 nm. The change in the absorbance of the quinone pigment at 555 nm is measured and quantified (formula (3)).

As described above, the reagents A, B, and C may be included in a reagent composition for quantifying a glycosylated protein, a glycosylated peptide, or a glycosylated dipeptide. The reagent composition may be in the form of, for example, a liquid, a frozen liquid, or a freeze dried product. The reagent for quantifying a glycosylated protein, a glycosylated peptide, or a glycosylated dipeptide may contain appropriate additives such as a surfactant, a salt, a buffer, a pH controlling agent, and an antiseptic.

The dipeptide-degrading enzyme is also useful for the measurement of a glycosylated protein, a glycosylated peptide, or a glycosylated dipeptide using FAO. The dipeptide-degrading enzyme degrades a glycosylated dipeptide which cannot or can hardly be degraded by a protease, thus enabling a glycosylated amino acid, which is a substrate for FAO, to be adequately fed. Then enzyme thus improves the insufficient accuracy of the prior art measurement of a glycosylated amino acid using a protease and FAO.

Examples

The present invention will be described below with reference to the following examples, but the present invention is not limited to these examples.

[Example 1] (Screening of dipeptide-degrading enzyme)

(1) Screening from microorganism

Various strains isolated from soil were cultured at 30°C for 24 hours in 1000 mL of a culture medium (pH 7.0) containing 10 g (1.0%) of Ehrlich's bonito extract, 10 g (1.0%) of polypeptone, 3 g (0.3%) of yeast extract, and 5 g (0.5%) of NaCl. The culture fluid thus obtained was centrifuged and separated into a culture supernatant and bacterial cells. The bacterial cells were washed, suspended in a 0.1M MOPS buffer (pH 7.0), fragmented by ultrasonic fragmentation, and thus the supernatant after centrifugation was obtained as a cell-free extract. The dipeptide-degrading activity of the culture fluid and cell-free extract obtained was examined in accordance with the method described in Example 3.

(2) Screening from commercial exogenous enzyme

A solution of a commercial protease was adjusted so as to give a nominal activity of 1 Ku/mL (when the protease was insoluble, it was adjusted so as to give a maximum concentration), and the dipeptide-degrading activity of the protease was measured in accordance with the method described in Example 3. It was found that none of the commercial proteases had dipeptide-degrading activity.

[Example 2] (Identification of dipeptide-degrading enzyme producing bacterium)

The dipeptide-degrading enzyme producing bacterium obtained by the screening in Example 1 was identified by the following method. The culture temperature was 30°C, except for the growth temperature test.
Incubation conditions: The bacterium was cultured for 3 days on a plate of TRIPTOSOY agar (Eiken Chemical Co., Ltd. hereinafter referred to as Eiken).
Morphology: The bacterium was cultured for one day on a plate of TRIPTOSOY agar (Eiken).
Gram staining: The bacterium was stained with FAVOR G set S (Nissui Pharmaceutical Co., Ltd., hereinafter referred to as Nissui Pharmaceutical).
Anaerobic culture: The bacterium was cultured for 3 days on a plate of TRIPTOSOY agar (Eiken). An anaerobic state was created with a GasPak anaerobic system (BBL).
Cytochrome oxidase: Cytochrome oxidase test paper (Nissui Pharmaceutical) was used.
Catalase: Bubble formation in 3% $H_2O_2$ was monitored.
O-F test: Mechanically sterilized glucose was added to OF Basal Medium (Difco) to give a concentration of 1%. A layer of liquid paraffin was formed on the culture medium to create an anaerobic state, where the bacterium was cultured for 7 days.
Nitrate reducibility: 0.1 % $KNO_3$ was added to Nutrient broth (Difco), and the bacterium was cultured therein for 10 days.
Citrate utilization: The bacterium was cultured for 14 days using Simmons Citrate agar (Eiken).
Urease: The bacterium was cultured for 14 days using Christensen's urea agar (Eiken).
Lecithinase: The bacterium was cultured for 5 days using 1000 mL of a culture medium containing 10 g of Bacto peptone, 1 g of glucose, 10 g of NaCl, 5 g of yeast extract (Difco), 15 g of agar, and 50 mL of egg yolk.
ONPG test (β-galactosidase): The bacterium was cultured for one day using ONPG disks (Nissui Pharmaceutical).
Acid formation from saccharide: To 1000 mL of a culture medium (pH 7.2) containing 1 g of $(NH_4)HPO_4$, 0.2 g of KCl, 0.2 g of $MgSO_4 \cdot 7H_2O$, 0.2 g of yeast extract (Difco), 15 g of Agar Noble (Difco), and 20 mL of 0.2% B.C.P, a sterilized sugar was added to give a concentration of 1%, and then the bacterium was cultured for 24 days using the culture medium.
Phenylalanine deaminase: The bacterium was cultured for 10 days using 1000 mL of a culture medium containing 2 g of DL-phenylalanine, 3 g of yeast extract (Difco), 1 g of $(NH_4)HPO_4$, 5 g of NaCl, and 15 g of agar.
Arginine dihydratase: The bacterium was cultured for 7 days using 1000 mL of a culture medium containing 10 g of Bacto-peptone (Difco), 5 g of NaCl, 0.3 g of $K_2HPO_4$, 10 g of L-arginine hydrochloride, 3 g of agar, and 20 mL of 0.2% phenol red. A layer of liquid paraffin was formed on the culture medium to create an anaerobic state.
Degradation of casein: The bacterium was cultured for 14 days using a culture medium containing Nutrient Agar (Eiken) and 5% skimmed milk (Difco).
Degradation of gelatin: The bacterium was cultured for 14 days using a culture medium containing Nutrient Agar (Eiken) and 12% gelatin.
Degradation of DNA: The bacterium was cultured for 14 days using a DNA agar (Nissui Pharmaceutical).
Degradation of Tween 80: The bacterium was cultured for 7 days using 1000 mL of a culture medium (pH 7.4) containing 10 g of Bacto-peptone (Difco), 5 g of NaCl, 0.1 g of $CaCl_2 \cdot 7H_2O$, 15 g of agar, and 10 g of Tween 80.
Degradation of tyrosine: The bacterium was cultured for 14 days using a culture medium containing Nutrient Agar (Eiken) and 0.5% tyrosine.
Degradation of esculin: The bacterium was cultured for 2 days using 1000 mL of a culture medium containing 10 g of Bacto-peptone (Difco), 0.5 g of iron citrate, and 1 g of esculin .
Degradation of starch: The bacterium was cultured for 14 days using a culture medium containing Nutrient Agar (Eiken) and 0.2% soluble starch.
Degradation of arbutin: The bacterium was cultured for 2 days using 1000 mL of a culture medium containing 5 g of arbutin, 5 g of yeast extract (Difco), and 15 g of Agar, added with 1% ferric ammonium citrate in the proportion of three drops to one petri dish (20 mL).
Growth on MacConkey agar: The bacterium was cultured for 14 days using MacConkey agar (Eiken).
Growth on cetrimide agar: The bacterium was cultured for 14 days using cetrimide agar (Nihon Pharmaceutical Co., Ltd.).
Growth temperature: The bacterium was cultured for 2 days or 7 days (10°C, 37°C, and 42°C) using a slant medium of TRIPTOSOY agar (Eiken).
Formation of 3-ketolactose: The bacterium was cultured for 14 days using 1000 mL of a culture medium containing 1 g of yeast extract (Difco), 10 g of lactose, and 15 g of agar.
Formation of indole: The bacterium was cultured for 14 days using 1000 mL of a culture medium containing 10 g of Tryptone (Difco).
API 20NE test: The test was carried out in accordance with the manufacturer's method. The bacterium was preincubated at 30°C for 2 days using TRIPTOSOY agar (Eiken).
Analysis of 16SrDNA base sequence: The bacterium was cultured at 30°C for 2 days using TRIPTOSOY agar (Eiken), and DNA preparation was carried out using DNeasy Tissue kit (QIAGEN). PCR and sequence reaction of the 16SrDNA were carried out using MicroSeq Full Gene 16SrDNA Kit (Applied Biosystems), and a dendrogram was prepared by multiple alignment and proximity junction.

The results of the identification are described below.

1. Morphological characteristics: The bacterium had a rod-like form with a size of 0.4 × 1.2 to 0.5 × 3.2 μm, and showed mobility.
2. Growth state: When the bacterium was grown on TRIPTOSOY agar, the form of the colonies was a projected circle having smooth edges, and the color of the colonies was light yellow. The bacterium did not grow on MacConkey agar or cetrimide agar.
3. Physiological properties: Listed in Table 1.
4. Genetical properties: Forms a cluster with Sphingopyxis chilensis in the dendrogram based on 16SrDNA.

[Table 1]

Table 1 Physiological properties of glycosylated dipeptide-degrading enzyme producing bacterium

| Evaluation item | Result |
|---|---|
| Gram staining properties | − |
| Anaerobic growth | − |
| Cytochrome C oxidase | + |
| Catalase | + |
| O-F test | Weak acid formation under aerobic conditions |
| Nitrate reducibility | − |
| Utilization of citrate | − |
| Utilization of malonate | − |
| Urease | − |
| Lecithinase | − |
| β -galactosidase | + |
| Indole production | − |
| Glucose acidification | − |
| Acid formation: | |
| Adonite | − |
| Arabinose | + |
| Cellobiose | + |
| Dulctiol | − |
| Glycerol | − |
| Inositol | + |
| Lactose | Weakly + |
| Maltose | + |
| Mannit | − |
| Raffinose | Weakly + |
| Rhamnose | − |
| Ribose | − |
| Salicin | Weakly + |
| Sorbit | − |
| Sucrose | + |
| Trehalose | + |
| Xylose | − |

| Evaluation item | Result |
|---|---|
| Phenylalanine deaminaze | − |
| Arginine dihydrolase | − |
| Casein degradation | − |
| Gelatine degradation | − |
| DNA degradation | − |
| Tween 80 degradation | + |
| Ttyrosine degradation | − |
| Esculin degradation | + |
| Starch degradation | + |
| Arbutin degradation | + |
| Culture temperature range | 10-37°C |
| 3-ketolactose formation | − |
| Indole formation | − |
| Utilization: | |
| Glucose | + |
| L-arabinose | − |
| D-mannose | + |
| D-mannitol | − |
| N-acetyl-D-glucosamine | − |
| Maltose | + |
| Potassium gluconate | − |
| n-capric acid | − |
| Adipic acid | − |
| dl-malic acid | + |
| Citric acid | − |
| Phenyl acetate | − |

−; Ngative
+; Positive

On the basis of the above results, the screened bacterium producing the dipeptide-degrading enzyme was identified as Sphingopyxis chilensis NO. 0522.

[Example 3] (Measurement of dipeptide-degrading activity)

The dipeptide-degrading activity may be measured by quantifying Fru-Val or histidine formed upon the degradation of Fru-Val-His. The method for quantifying histidine is described below. The amount of enzyme degrading 1 μmol of Fru-Val-His in 1 minute is defined as 1 unit of enzymatic activity (U). The reagents (1), (2), and (3), and a substrate solution used for measuring the activity were prepared as described below.

| | |
|---|---|
| Reagent (1): PIPES pH 6.2 (Dojindo Laboratories) | 100 mM |
| $CoCl_2$ (Wako Pure Chemical Industries, Ltd.) | 2 mM |

(continued)

| | |
|---|---|
| Triton X-100 (Sigma-Aldrich Japan K.K.) | 0.1% |
| Regent (2): | |
| MOPS pH 7.5 (Dojindo Laboratories) | 300 mM |
| 4-AA (Wako Pure Chemical Industries, Ltd.) | 2 mM |
| EDTA (Dojindo Laboratories) | 5mM |
| Triton X-100 (Sigma) | 0.1% |
| $NaN_3$ (Wako Pure Chemical Industries, Ltd.) | 0.1% |
| L-amino acid oxidase (Wako Pure Chemical Industries, Ltd.) | 10 U/mL |
| Peroxidase (PO-3, Amano Enzyme Inc.) | 14.3 U/mL |
| Reagent (3): | |
| Reagent (2) and 20 mM phenol were mixed at a ratio of 7:1, and stored away from light. | |

Substrate solution: Fru-Val-His (Peptide Institute Inc.) was dissolved in 100 mM of a MOPS buffer (pH 7.0), thus obtaining 40 mM of substrate solution.

0.02 mL of 1.40 mM substrate solution and 0.16 mL of the reagent (1) were accurately dispensed and mixed, and preincubated at 37°C for 5 minutes. Thereafter, 0.02 mL of enzyme solution was accurately added to and mixed with the mixture, and allowed to react at 37°C. As a blank test, 0.02 mL of the reagent (1) was added in place of the enzyme solution. After a lapse of 10 minutes, 0.8 mL of the reagent (3) was accurately added to and mixed with the mixture, and allowed to react at 37°C. After a lapse of 15 minutes, the absorbance at 505 nm was measured, and the dipeptide-degrading activity was calculated according to the calculation formula described below. The enzyme solution was appropriately diluted with the reagent (1) such that ΔABS = (As-Ab) ≤ 0.50 Abs, and used as the sample.
The symbols and numerical values in the calculation formula represents the followings.

As: absorbance of enzyme solution
Ab: absorbance of blank solution
ΔABS: As - Ab
12.88: millimole absorptivity (mmol/L/cm) of quinone pigment at 505 nm
10: reaction time (min)
2: coefficient representing that one molecule of quinone pigment is formed from two molecules of $H_2O_2$
1: light path length (cm)
1.0: total amount of reaction solution (mL)
0.02: Amount of enzyme solution used for reaction (mL)
n: enzyme solution dilution ratio

Calculation formula

$$\text{Activity (U/mL)} = \Delta\text{ABS}/10/12.88 \times 2 \times 1 \times 1.0/0.02 \times n$$

[Example 4] (Preparation of crude enzyme solution)

Sphingopyxis chilensis No. 0522 was cultured at 28°C for 36 hours in 1000 mL of a culture medium (pH 7.0) containing 10 g (1.0%) of Ehrlich's bonito extract, 10 g (1.0%) ofpolypeptone, 3 g (0.3%) of yeast extract, and 5 g (0.5%) of NaCl. Since the dipeptide-degrading enzyme is an intracellular enzyme, the culture fluid was centrifuged, thus collecting the bacterial cells. The bacterial cells were washed, suspended in a 0.1M MOPS buffer (pH 7.0), fragmented by ultrasonic fragmentation, and thus the supernatant after centrifugation was obtained as a cell-free extract.

[Example 5] (Preparation of partially purified enzyme)

The above-described cell-free extract was salted out with 30% to 50% saturated ammonium sulfate, and centrifuged to obtain a deposit. The deposit was dissolved in a 30 mM MOPS buffer (pH 7.0), and then demineralized and concentrated using an ultrafiltration membrane (trade name MICROZA, molecular cutoff 3000, Asahi Kasei Corporation, hereinafter the same). The concentrate thus obtained was passed through an anion exchange resin column (trade name: DEAE-Sepharose CL-6B, GE Healthcare Bio-Sciences K.K.), which had been equilibrated with a 30 mM MOPS buffer (pH 7.0), thereby allowing the dipeptide-degrading enzyme adsorbed thereto, and the column was washed with the same buffer. Subsequently, the dipeptide-degrading enzyme was eluted with a 30 mM MOPS buffer (pH 7.0) and another 30 mM MOPS buffer (pH 7.0) containing 0.2M NaCl by a linear gradient elution method in a NaCl concentration range of 0 to 0.2 M, thus a partially purified enzyme was obtained.

[Example 6] (Isolation of single enzyme)

The solution of the partially purified enzyme obtained above was concentrated using an ultrafiltration membrane, and charged into a gel filtration chromatography column (trade name: Hi Load 16/60 Superdex 200pg, GE Healthcare Bio-Sciences K.K.) equilibrated with a 30 mM MOPS buffer (pH 7.0) containing 0.2 M NaCl. The solution was fractionated at a flow rate of 0.5 mL/min, and the active fraction was collected. The purified enzyme solution thus obtained was subjected to Native-PAGE, placed on an agarose gel containing a reagent for measuring the enzymatic activity, and then activity staining was carried out using the dipeptide-degrading activity as the index. Subsequently, the band showing activity was cut out, and the enzyme was extracted therefrom. In the analysis of the purity of the extract by SDS-PAGE, a single band was detected in the vicinity of 45 kDa (Fig. 5), thus obtaining a purified dipeptide-degrading enzyme.

[Example 7] (Physicochemical properties of dipeptide-degrading enzyme)

The physicochemical properties of the purified dipeptide-degrading enzyme obtained above were studied as follows.

1. Study on optimum pH and pH stability

In order to determine the optimum pH, the procedure in Example 3 was carried out, except that the buffer of the reagent (1) described in Example 3 was replaced with various buffers (0.1 M acetic acid buffer with pH 4 to 6, 0.1 M PIPES buffer with pH 6 to 7.5, and 0.1 M TES buffer with pH 7.5 to 8, and 0.1 M Tris buffer with pH 8 to 9), thereby adjusting the pH to 5 to 9. Further, in order to examine the pH stability, the dipeptide-degrading enzyme was incubated at 37°C for 1 hour in various buffers (0.1 M acetic acid buffer with pH 4 to 6, 0.1 M MES buffer with pH 6 to 6.5, 0.1 M MOPS buffer with pH 6.5 to 7.5, 0.1 M TES buffer with pH 7.5 to 8.0, and Tris buffer with pH 8.0 to 9.0), and then the activity was measured in the same manner as in Example 3 (37°C, pH 6.2).

The results indicate that the optimum pH of the dipeptide-degrading enzyme is 6.2, and the activity of the enzyme in the pH range of 6.0 to 7.0 is 80% or more of the activity at pH 6.2 (Fig. 1), and that the enzyme is stable at pH 5.0 or higher (Fig. 2).

2. Study on optimum temperature and temperature stability

In order to determine the optimum temperature, the procedure in Example 3 was carried out, except that the temperature during the first reaction was changed in the range of 20 to 50°C. Further, in order to examine the temperature stability, the dipeptide-degrading enzyme was dissolved in a 0.1 M MOPS buffer (pH 7.0), incubated at 20 to 50°C for 1 hour, and then the enzymatic activity was measured in the same manner as in Example 3. The results indicate that the optimum temperature of the dipeptide-degrading enzyme is 45°C, and the enzymatic reaction adequately proceeds in the temperature range of 35 to 45°C (Fig. 3), and that the enzyme stably keeps its activity at 50°C or lower, suggesting that the enzyme has high temperature stability (Fig. 4).

3. Molecular weight (SDS-PAGE)

Using PhastGel Gradient 10-15 (GE Healthcare Bio-Sciences K.K.), SDS-PAGE was carried out with a Phast-Systemfull automatic electrophoresis apparatus (GE Healthcare Bio-Sciences K.K.) in accordance with the instruction of Phast-System. As a standard protein, LMW Marker Kit (GE Healthcare Bio-Sciences K.K.) was subjected to electrophoresis in the same manner, thus determining the molecular weight. The molecular weight was about 45 kDa.

4. Influence of metal ions and the like on enzymatic activity

The enzymatic activity was measured in the same manner as Example 3, except that the reagent (1) was replaced with various metals and the like at specified concentrations (Table 2) and a 100 mM PIPES buffer (pH 6.2) containing 0.1% Triton X-100. Table 2 shows the influences of the metal ions and the like on the enzymatic activity, indicating that the enzyme is activated by $Mg^{2+}$, $Co^{2+}$, and $Zn^{2+}$ ions, and deactivated by EDTA and $\alpha,\alpha'$-dipyridyl.

[Table 2]

| Compound | Conc. | Relative activity (%) |
|---|---|---|
| None | | 100 |
| $CaCl_2$ | 1mM | 162 |
| $MgCl_2$ | 1mM | 261 |
| $MnCl_2$ | 1mM | 137 |
| $ZnCl_2$ | 1mM | 257 |
| $BaCl_2$ | 1mM | 105 |
| $CoCl_2$ | 1mM | 676 |
| $CuCl_2$ | 1mM | 75 |
| $NiCl_2$ | 1mM | 156 |
| $NH_4Cl$ | 50mM | 85 |
| NaCl | 50mM | 98 |
| KCl | 50mM | 97 |
| EDTA | 5mM | 1 |
| SDS | 2mM | 9 |
| Thiourea | 2mM | 104 |
| Sodium Hexametaphosphate | 2mM | 4 |
| Monoiode acetate | 2mM | 111 |
| Thioglycolic acid | 2mM | 3 |
| 2-Mercaptoethanol | 2mM | 11 |
| o-Phenanthroline | 2mM | 0 |
| $\alpha$, $\alpha$'-Dipyridyl | 2mM | 1 |
| Pepstatin A | 10 $\mu$g/ml | 100 |
| PMSF | 2mM | 97 |

[Example 8] (Determination of sequence of N terminals and internal amino acids of purified enzyme)

The sequence of the N terminals and internal amino acids of the purified dipeptide-degrading enzyme obtained above was analyzed at Proteomic Analysis Center, Shimadzu Co., Ltd., thus obtaining three partial amino acid sequences indicated by the sequence numbers 3, 4, and 5.

Example 9] (Designing of oligonucleotide primer)

[On the basis of the base sequence deduced from No. 3 and No. 4 amino acid sequences, the primers 6 and 7 used for polymerase chain reaction (PCR) were designed. The base sequence of the primers are indicated with sequence No. 6 and No. 7.

[Example 10] (Preparation of chromosomal DNA)

Sphingopyxis chilensis No. 0522 was cultured at 30°C for 16 hours in 1000 mL of a culture medium (pH 7.0) containing 10 g (1.0%) of Ehrlich's bonito extract, 10 g (1.0%) of polypeptone, 3 g (0.3%) of yeast extract, and 5 g (0.5%) of NaCl, and then the bacterial cells were collected by centrifugation. The bacterial cells were suspended in 100 mM EDTA (pH 8.0) containing 150 mM NaCl, and Lysozyme (Roche Diagnostics K.K.) and Rnase A (Sigma-Aldrich Japan K.K.) were added thereto in the proportions of 4 mg /mL and 20 $\mu$g/mL, respectively. The culture medium was further incubated at 37°C for 1 hour.
Subsequently, a 100 mM Tris-hydrochloride buffer (pH 9.0) containing 3 volumes of 1 % SDS was uniformly mixed with the culture medium. The mixture was further mixed with an equal amount of TE saturated phenol. The mixture was centrifuged, and the supernatant was collected. The supernatant was mixed with 0.6 volume of isopropanol, and the mixture was allowed to stand at -20°C for 2 hours. After centrifugation, the supernatant was discarded, and the precipitate was washed with 70% ethanol and dried in vacuo, thereby obtaining the chromosomal DNA of Sphingopyxis chilensis No. 0522.

[Example 11] (Probe preparation by PCR)

PCR was carried out using the primers 6 and 7, and the above-described chromosomal DNA. The chromosomal DNA adjusted to 1 mg /mL was diluted with 10 volumes of a TE buffer, and 1 μL of the dilution was mixed with the following solution.

| | |
|---|---|
| LA Taq (5 U/μL) (Takara Bio Inc.) | 0.5 μL |
| GC buffer I (Takara Bio Inc.) | 25 μL |
| dNTP mix | 8 μL |
| Primer 6 | 1 μL |
| Primer 7 | 1 μL |
| Sterilized water | 13.5 μL |

The mixture was subjected to 30 cycles of heat treatment consisted of 96°C for 30 seconds, 60°C for 30 seconds, and 72°C for 1 minute, and then heated at 72°C for 5 minutes, thereby accomplishing the PCR. Subsequently, the mixture was subjected to agarose electrophoresis, confirming that a fragment of about 500 bp was amplified by the PCR.

[Example 12] (Subcloning of PCR fragment)

The fragment of about 500 bp amplified by the above-described PCR was cut out from the agarose gel, and purified using TaKaRa Easy Trap Kit (Takara Bio Inc.). The fragment was phosphorylated with T4 kinase, and then TA cloned using pGEM T Easy vector System (Promega K.K.). The base sequence of the plasmid inserted with the PCR fragment was determined using 370 DNA Sequencing System (Applied Biosystems) (sequence No. 8). On the basis of the base sequence, primers 9 and 10 were designed (sequence No. 9 and No. 10), and PCR was carried out using the above-described chromosomal DNA. A 1 mg/mL chromosomal DNA solution was diluted with 10 volumes of a TE buffer, and 1 μL of the dilution was mixed with the following solution.

| | |
|---|---|
| LA Taq (5 U/μL) (Takara Bio Inc.) | 0.5 μL |
| GC buffer I (Takara Bio Inc.) | 25 μL |
| dNTP mix | 8 μL |
| Primer 9 | 1 μL |
| Primer 10 | 1 μL |
| Sterilized water | 13.5 μL |

The mixture was subjected to 30 cycles of heat treatment consisted of 96°C for 30 seconds, 60°C for 30 seconds, and 72°C for 1 minute, and then heated at 72°C for 5 minutes, thereby accomplishing the PCR. Subsequently, the mixture was subjected to agarose electrophoresis, a fragment of about 500 bp was cut out from the agarose gel, and purified using TaKaRa Easy Trap Kit (Takara Bio Inc.). The PCR fragment thus obtained was labeled with digoxigenin using DIG High Prime (Roche Diagnostics K.K.).

[Example 13] (Southern hybridization)

Southern hybridization was carried out using the above-described probe (sequence No. 8). Chromosomal DNAs completely digested with HindIII, SphI, PstI, XbaI, BamHI, SmaI, KpnI, SacI, EcoRI, and SalI, and combinations of these restriction enzymes and EcoRV were separated by 0.8% agarose electrophoresis, and transferred onto Zeta-Probe membrane (Bio-Rad Laboratories, Inc.) in 0.4 M NaOH, thus forming a membrane for southern hybridization. The procedure of the southern hybridization is as follows. Hybridization was carried out using DIG Easy Hyb (Roche Diagnostics K.K.) at 42°C for 16 hours, the hybrid was washed twice for 5 minutes each in 2 × SSC containing 0.1% SDS at room temperature, and washed for 5 minutes in 0.1 × SSC containing 0.1% SDS at 68°C. The hybrid was detected with a digoxigenin antibody labeled with alkaline phosphatase, and a restriction enzyme map around the intended gene was prepared based on the result (Fig. 6). The restriction enzyme map indicates that the fragment of 2.4 Kbp completely digested with BamHI and EcoRI contains the intended gene. Therefore, the chromosomal DNA completely digested with BamHI and EcoRI was subjected to 0.8% agarose electrophoresis, and then the fragment in the vicinity of 2.4 Kbp was cut out from the agarose, and inserted into the BamHI and EcoRI sites of pUC 18 (Takara Bio Inc.) plasmid. 1000 strains of Escherichia coli JM109 (Takara Bio Inc.) transformed with the recombinant plasmid were prepared. The colonies of the transformant thus obtained were transferred to a nylon membrane (Roche Diagnostics K.K.), and alkali fixed in 0.5M NaOH containing 1.5 M NaCl, thus making a membrane for colony hybridization. Subsequently, hybridization was carried out using DIG Easy Hyb (Roche Diagnostics K.K.) at 42°C for 16 hours, the hybrid was washed twice for 5 minutes each in 2 × SSC containing 0.1% SDS at room temperature, and further washed for 5 minutes in 0.1 × SSC containing 0.1% SDS at 68°C. The hybrid was detected with a digoxigenin antibody labeled with alkaline phosphatase, and several positive clone strains were obtained. Plasmids were collected from the clone, and the base sequence was determined using 370 DNA Sequencing System (Applied Biosystems). The base sequence of the dipeptide-degrading enzyme gene thus determined is indicated with sequence No. 1, and the amino acid sequence of the polypeptide translated from the DNA sequence is indicated with sequence No. 2. The gene has a coding region of 1374 bp, and codes for 457 amino acid residues. The amino acid sequence deducted from the base sequence contains the N-terminal and internal amino acid sequences of the purified enzyme determined in Example 8. The fact suggests that the recombinant plasmids thus obtained contain a gene coding for the dipeptide-degrading enzyme.

On the basis of the base sequence of the dipeptide-degrading enzyme gene revealed in the above example, primers 11 and 12 were constructed (sequence No. 11 and No. 12), and PCR was carried out using the chromosomal DNA described in Example 10. The chromosomal DNA solution adjusted to 1 mg/mL was diluted with 10 volumes of a TE buffer, and 1 μL of the dilution was mixed with the following solution.

| | |
|---|---|
| LA Taq (5 U/μL) (Takara Bio Inc.) | 0.5 μL |
| GC buffer I (Takara Bio Inc.) | 25 μL |
| dNTP mix | 8 μL |
| Primer 11 | 1 μL |
| Primer 12 | 1 μL |
| Sterilized water | 13.5 μL |

The mixture was subjected to 30 cycles of heat treatment consisted of 96°C for 30 seconds, 60°C for 30 seconds, and 72°C for 1 minute, and then heated at 72°C for 5 minutes, thereby accomplishing the PCR. Subsequently, the mixture was subjected to agarose electrophoresis, a PCR fragment of about 1400 bp was cut out from the agarose gel, and purified using TaKaRa Easy Trap Kit (Takara Bio Inc.). The gene fragment thus obtained was treated with restriction enzymes PagI and SalI, and linked to pTrc99a (GE Healthcare Bio-Sciences K.K.) treated with restriction enzymes NcoI and SalI. The plasmid was named pTPRGA. Using the pTPRGA, Escherichia coli JM109 was transformed, thus obtaining an Escherichia coli JM109 transformant (pTPRGA).

[Example 14] (Production of enzyme by transformant, activity measurement, and partial purification of enzyme)

Escherichia coli JM109 (pTPRGA) was cultured at 37°C for 20 hours while shaking in 100 mL of a LB medium (1% Bacto Tryptone, 0.5% Bacto Yeast extract, 1% sodium chloride, pH 7.0) containing 100 μg/mL ampicillin and 0.2 mM isopropyl-β-D(-)-thiogalactopyranoside (IPTG). Thereafter, the bacterial cells were collected by centrifugation, suspended in 10 mL of a 100 mM MOPS buffer (pH 7.0), and fragmented by ultrasonic fragmentation. The solution was centrifuged, and the supernatant was obtained as a cell-free extract. The dipeptide-degrading activity of the extract was about 0.05 U/mL.

As described above, the dipeptide-degrading enzymatic activity was found in the cell-free extract prepared from the culture fluid of Escherichia coli JM109 (pTPRGA), indicating that the fragment inserted into the pTPRGA is a dipeptide-degrading enzyme gene.

A cell-free extract of Escherichia coli JM109 (pTPRGA) was salted out with 30% to 50% saturated ammonium sulfate, and the deposit was collected. The deposit was dissolved in a 30 mM MOPS buffer (pH 7.0), and then demineralized and concentrated using an ultrafiltration membrane. The dipeptide-degrading enzyme contained in the concentrate was allowed to be adsorbed to an anion exchange resin column (trade name: DEAE-Sepharose CL-6B, GE Healthcare Bio-Sciences K.K.), which has been equilibrated with a 30 mM MOPS buffer (pH 7.0), and washed with the same buffer. Subsequently, the dipeptide-degrading enzyme was eluted with a NaCl-containing 30 mM MOPS buffer (pH 7.0) by a linear gradient elution method in a NaCl concentration range af 0 to 0.2 M, thus a partially purified recombinant dipeptide-degrading enzyme was obtained.

[Example 15] (Measurement of glycosylated dipeptide using dipeptide-degrading enzyme)

A glycosylated dipeptide was measured using a glycosylated hemoglobin β chain dipeptide Fru-Val-His (Peptide Institute Inc.). The reagents B and C were prepared according to the following recipes, and used for the study. Since the specimen was a glycosylated dipeptide, no protease was necessary. Therefore, the reagent E used herein had the same composition with the reagent A except that no protease was used. However, the glycosylated dipeptide may be quantified only with the reagents B and C without using the reagent E. The dipeptide-degrading enzyme used herein is the purified enzyme obtained in Example 6.

| | |
|---|---|
| Reagent E: MES pH 6.0 (Dojindo Laboratories) | 100 mM |
| $CaCl_2 \cdot 6H_2O$ | 2 mM |
| Triton X-100 (Sigma-Aldtich Japan K.K.) | 0.1% |
| $NaN_3$ (Wako Pure Chemical Industries, Ltd.) | 0.02% |
| Reagent B: | |
| MOPS pH 7.5 (Dojindo Laboratories) | 100 mM |
| Triton X-100 (Sigma-Aldrich Japan K.K.) | 0.1% |
| $NaN_3$ (Wako Pure Chemical Industries, Ltd.) | 0.02% |
| 4-AA (Wako Pure Chemical Industries, Ltd.) | 1.05 mM |
| L-amino acid oxidase (Wako Pure Chemical Industries, Ltd.) | 50 U/mL |
| Catalase (Amano Enzyme Inc.) | 5 U/mL |
| Reagent C: | |
| PIPES pH 7.0 (Dojindo Laboratories) | 300 mM |
| Triton X-100 (Sigma-Aldrich Japan K.K.) | 0.1% |
| $NaN_3$ (Wako Pure Chemical Industries, Ltd.) | 0.1% |
| Dipeptide-degrading enzyme (purified product) | 2.4 U/mL |
| $CoCl_2$ (Wako Pure Chemical Industries, Ltd.) | 2 mM |
| TOOS (Dojindo Laboratories) | 5.835 mM |
| Peroxidase (PO-3, Amano Enzyme Inc.) | 16.5 U/mL |
| EDTA (Dojindo Laboratories) | 5mM |

40 mM Fru-Val-His was prepared, and diluted with a 100 mM MOPS buffer (pH 7.0), thus making 0.1, 0.2, 0.4, 0.6, and 0.8 mM Fru-Val-His solutions. To 0.01 mL each of the diluting solutions, 0.04 mL of the reagent E was added, and allowed to react at 37°C for 5 minutes. 0.1 mL of the reagent B was added to each of the reaction liquids, and allowed to react at 37°C for 10 minutes. Thereafter, 0.9 mL of the reagent C was added to each of the reaction liquids, and allowed to react at 37°C. The absorbance at 555 nm was measured, and the increase (ΔOD) of the absorbance 5 minutes after initiation of the reaction was determined. Fig. 7 shows the measurements of Fru-Val-His at different concentrations. Fig. 7 indicates that the concentration of Fru-Val-His in the sample is linearly correlated with the increase of the absorbance. The fact suggests that the dipeptide-degrading enzyme degrades Fru-Val-His into Fru-Val and L-histidine, and that the Fru-Val-His in the sample is quickly and accurately measured by the present method.

Fru-VHLTPE, which is a glycosylated hemoglobin β chain peptide, may be used for the measurement of a glycosylated peptide. In this case, the reagents A, B, and C were prepared according to the following recipe. The Fru-VHLTPE is degraded by the reagent A, and the Fru-Val-His thus produced is measured using the reagents B and C.

| | |
|---|---|
| Reagent A: MES pH 6.0 (Dojindo Laboratories) | 100 mM |
| $CaCl_2 \cdot 6H_2O$ | 2 mM |
| Triton X-100 (Sigma-Aldrich Japan K.K.) | 0.1 % |
| $NaN_3$ (Wako Pure Chemical Industries, Ltd.) | 0.02% |
| Protease (thermolysin, Daiwa Fine Chemicals Co., Ltd.) | 250 U/mL |
| Reagent B: | |
| MOPS pH 7.5 (Dojindo Laboratories) | 100 mM |
| Triton X-100 (Sigma-Aldrich Japan K.K.) | 0.1% |
| $NaN_3$ (Wako Pure Chemical Industries, Ltd.) | 0.02% |
| 4-AA (Wako Pure Chemical Industries, Ltd.) | 1.05 mM |
| L-amino acid oxidase (Wako Pure Chemical Industries, | Ltd.) 50 U/mL |
| Catalase (Amano Enzyme Inc.) | 5 U/mL |
| Reagent C: | |
| PIPES pH 7.0 (Dojindo Laboratories) | 300 mM |

(continued)

| | |
|---|---|
| Reagent C: | |
| TritonX-100 (Sigma-Aldrich Japan K.K.) | 0.1% |
| $NaN_3$ (Wako Pure Chemical Industries, Ltd.) | 0.1 % |
| Dipeptide-degrading enzyme (purified product) | 2.4 U/mL |
| $CoCl_2$ (Wako Pure Chemical Industries, Ltd.) | 2 mM |
| TOOS (Dojindo Laboratories) | 5.835 mM |
| Peroxidase (PO-3, Amano Enzyme Inc.) | 16.5 U/mL |
| EDTA (Dojindo Laboratories) | 5 mM |

[Example 16] (Study on substrate specificity of dipeptide-degrading enzyme)

In order to examine the substrate specificity of the dipeptide-degrading enzyme, the substrate used for the measurement of the dipeptide-degrading activity in Example 3 was changed from the glycosylated hemoglobin β chain dipeptide Fru-Val-His to a glycosylated hemoglobin α chain dipeptide Fru-Val-Leu (fructosyl valyl leucine) (Peptide Institute Inc.), and the reactivity was tested. As a result of this, the activity value of the dipeptide-degrading enzyme for the Fru-Val-Leu substrate was 4.9% of that for Fru-Val-His. Thereafter, the reactivity in an actual measurement system was tested using 0.8 mM Fru-Val-His and 0.8 mM Fru-Val-Leu as substrates in accordance with the method in Example 15 for measuring a glycosylated dipeptide using the dipeptide-degrading enzyme. The results are shown in Fig. 8. The absorbance change for the Fru-Val-Leu substrate shown in Fig. 8 was converted into the Fru-Val-His concentration using Fig. 7. As a result of this, the Fru-Val-His concentration was 0.14 mM, corresponding to 18% reactivity for the 0.8 mM substrate. The fact suggests that the reactivity of the dipeptide-degrading enzyme for the glycosylated hemoglobin α chain dipeptide Fru-Val-Leu is low. Accordingly, the measurement of a glycosylated hemoglobin using the dipeptide-degrading enzyme of the present invention has high specificity for glycosylated hemoglobin β chain proteins, and thus allows highly accurate measurements. In Fig. 8, Fru-VH represents Fru-Val-His, and Fru-VL represents Fru-Val-Leu.

**Claims**

1. A dipeptide-degrading enzyme which acts on a dipeptide to produce amino acids.

2. The dipeptide-degrading enzyme according to claim 1, wherein the dipeptide is a glycosylated dipeptide, and a glycosylated amino acid and an amino acid are produced.

3. The dipeptide-degrading enzyme according to claim 2, wherein the glycosylated dipeptide is glycosylated Val-His which is a glycosylated hemoglobin β chain dipeptide, the glycosylated amino acid is glycosylated Val, and the amino acid is histidine.

4. A dipeptide-degrading enzyme having the following physicochemical properties:

(1) Action and substrate: The dipeptide-degrading enzyme acting on a dipeptide to produce amino acids, and acting on a glycosylated dipeptide to produce a glycosylated amino acid and an amino acid.
(2)Optimum pH: The optimum pH of the dipeptide-degrading enzyme is 6.2, and the activity of the enzyme in the pH range of 6.0 to 7.0 is 80% or more of the activity at pH6.2.
(3) pH stability: The dipeptide-degrading enzyme is stable at pH 5.0 or higher.
(4) Optimum temperature: The optimum temperature of the dipeptide-degrading enzyme is at 45°C, and the enzymatic reaction adequately proceeds in the temperature range of 35 to 45°C.
(5) Heat stability: The dipeptide-degrading enzyme stably keeps its activity at 50°C or lower.
(6) Molecular weight: The molecular weight of the dipeptide-degrading enzyme is about 45k Da (SDS-PAGE).
(7) Activator and inhibitor: The dipeptide-degrading enzyme is activated by $Mg^{2+}$, $Co^{2+}$, or $Zn^{2+}$ ions, and inhibited by EDTA or α,α'-dipyridyl.

5. The dipeptide-degrading enzyme according to any one of claims 1 to 4, which is derived from a microorganism belonging to genus Sphingopyxis.

6. The dipeptide-degrading enzyme according to claim 5, wherein the microorganism belonging to genus Sphingopyxis is derived from Sphingopyxis chilensis No. 0522 (NITE BP-250) or a mutant strain thereof.

7. A protein (a) or (b):

(a) a protein having an amino acid sequence indicated by SEQ ID No. 2 in the sequence listing;
(b) a protein consisting of an amino acid sequence obtained by deletion, substitution, or addition of one or several amino acids in the amino acid sequence indicated by SEQ ID No. 2 in the sequence listing, and having dipeptide-degrading activity.

**8.** A gene coding for the following protein (a) or (b):

(a) a protein having an amino acid sequence indicated by SEQ ID No. 2 in the sequence listing;
(b) a protein having an amino acid sequence obtained by deletion, substitution, or addition of one or several amino acids in the amino acid sequence indicated by SEQ ID No. 2 in the sequence listing, and having dipeptide-degrading activity.

**9.** A gene comprising the following DNA (a), (b), or (c):

(a) DNA having a base sequence indicated by SEQ ID No. 1 in the sequence listing;
(b) DNA having a base sequence obtained by deletion, substitution, or addition of one or several bases in the base sequence indicated by SEQ ID No. 1 in the sequence listing, and coding for a protein having dipeptide-degrading activity; or
(c) DNA which hybridizes with a DNA having the base sequence indicated by SEQ ID No. 1 in the sequence listing under stringent conditions, and codes for a protein having dipeptide-degrading activity.

**10.** A recombinant vector comprising the gene according to claim 8 or 9.

**11.** A transformant or transductant obtained by transformation of a host cell by the recombinant vector according to claim 10.

**12.** The transformant or transductant according to claim 11, wherein the host cell is Escherichia coli or a microorganism belonging to genus Pseudomonas.

**13.** A method for producing a dipeptide-degrading enzyme, comprising culturing a microorganism belonging to genus Sphingopyxis, allowing the dipeptide-degrading enzyme according to any one of claims 1 to 4 to be produced, and collecting the dipeptide-degrading enzyme.

**14.** The method for producing a dipeptide-degrading enzyme according to claim 13, wherein the microorganism belonging to genus Sphingopyxis is derived from Sphingopyxis chilensis No. 0522 (NITE BP-250) or a mutant strain thereof.

**15.** Sphingopyxis chilensis No. 0522 (NITE BP-250) which is a microorganism producing the dipeptide-degrading enzyme according to any one of claims 1 to 4.

**16.** A method for producing a glycosylated amino acid and an amino acid, comprising allowing the dipeptide-degrading enzyme according to any one of claims 1 to 4 or the protein according to claim 7 to act on a specimen containing a glycosylated dipeptide.

**17.** The method for producing a glycosylated amino acid and an amino acid according to claim 16, wherein the glycosylated dipeptide is glycosylated Val-His which is a glycosylated hemoglobin β chain dipeptide.

**18.** A method for producing a glycosylated amino acid and an amino acid, comprising allowing a protease and the dipeptide-degrading enzyme according to any one of claims 1 to 4 or the protein according to claim 7 to act on a specimen containing a glycosylated protein or a glycosylated peptide.

**19.** The method for producing a glycosylated amino acid and an amino acid according to claim 18, wherein the glycosylated protein is a glycosylated hemoglobin β chain protein, and the glycosylated peptide is a degradation product of a glycosylated hemoglobin β chain protein.

**20.** A method for measuring a glycosylated protein, a glycosylated peptide, or a glycosylated dipeptide, comprising allowing amino acids produced by the method according to any one of claims 16 to 19 to be degraded by a substance which degrades the amino acids, and quantifying the amino acids.

**21.** The method for measuring a glycosylated protein, a glycosylated peptide, or a glycosylated dipeptide according to claim 20, wherein the substance which degrades the amino acid is a dehydrogenase or an oxidase.

**22.** The method for measuring a glycosylated protein, a glycosylated peptide, or a glycosylated dipeptide according to claim 21, wherein the oxidase is an amino acid oxidase.

**23.** A method for measuring a glycosylated protein, a glycosylated peptide, or a glycosylated dipeptide, comprising using an oxidase to quantify the glycosylated amino acid produced by the method according to any one of claims 16 to 19.

**24.** The method for measuring a glycosylated protein, a glycosylated peptide, or a glycosylated dipeptide according to claim 23, wherein the oxidase is a fructosyl amino acid oxidase.

**25.** A reagent composition to be used for carrying out the measurement method according to any one of claims 20 to 24.

**26.** The reagent composition according to claim 25, comprising at least reagents B and C, and optionally a reagent A, the reagent A containing a substance having protease activity, the reagent B containing a substance which eliminates an amino acid produced by the protease activity of the reagent A, and the reagent C containing the dipeptide-degrading enzyme according to any one of claims 1 to 4 or the protein according to claim 7, and a substance which degrades the glycosylated amino acid or amino acid degraded by the dipeptide-degrading enzyme or the protein.

**27.** The reagent composition according to claim 25, comprising at least reagents B, C, and D, and optionally a reagent A, the reagent A containing a substance having protease activity, the reagent B containing a substance which eliminates an amino acid produced by the protease activity of the reagent A, the reagent C containing the dipeptide-degrading enzyme according to any one of claims 1 to 4 or the protein according to claim 7, and the reagent D containing a substance which degrades the glycosylated amino acid or amino acid degraded by the dipeptide-degrading enzyme or the protein.

Fig.1

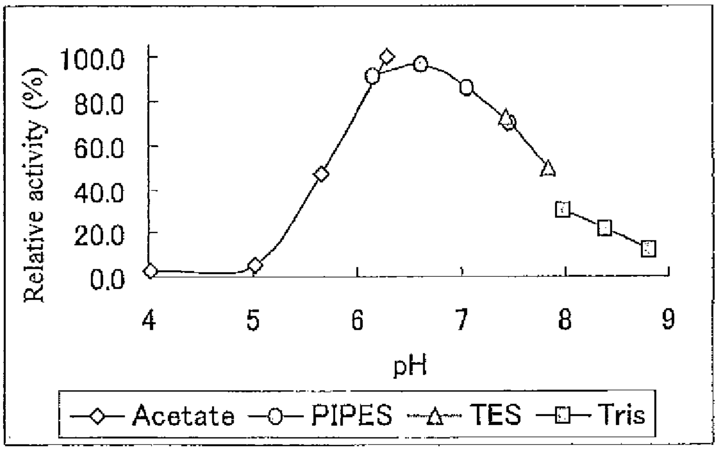
Relative activity (%)
100.0
80.0
60.0
40.0
20.0
0.0
4
5
6
7
8
9
pH
Acetate
PIPES
TES
Tris

Fig.2

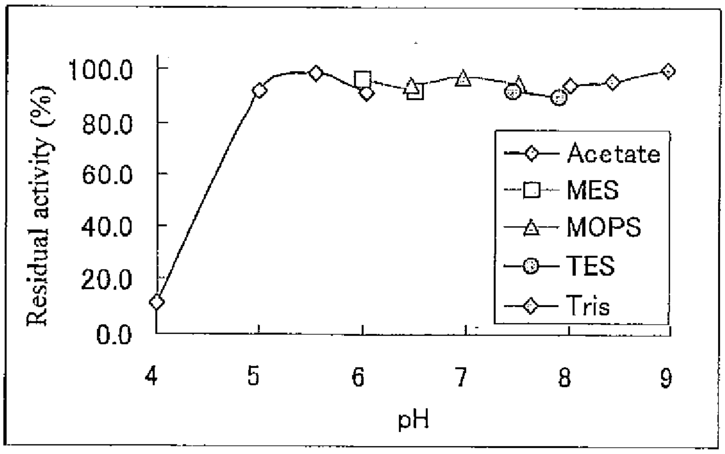
100.0
80.0
60.0
40.0
20.0
0.0
Residual activity (%)
4
5
6
7
8
9
pH
Acetate
MES
MOPS
TES
Tris

Fig.3

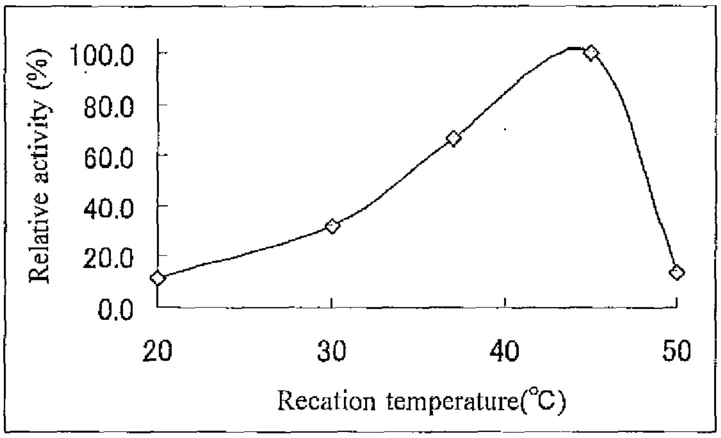
Relative activity (%)
100.0
80.0
60.0
40.0
20.0
0.0
20
30
40
50
Recation temperature(°C)

Fig.4

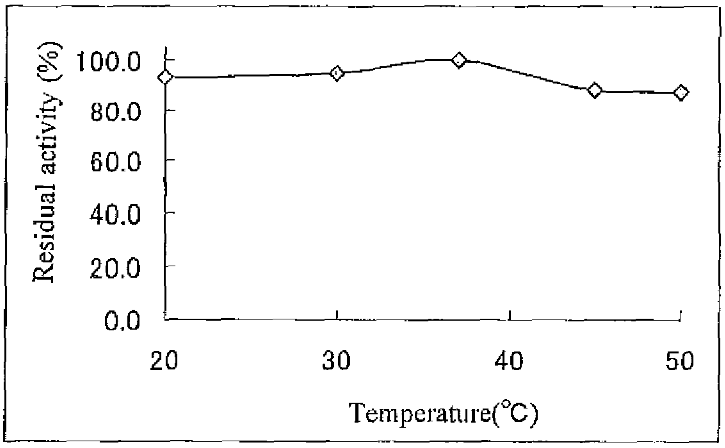
Residual activity (%)
100.0
80.0
60.0
40.0
20.0
0.0
20
30
40
50
Temperature(°C)

Fig.5

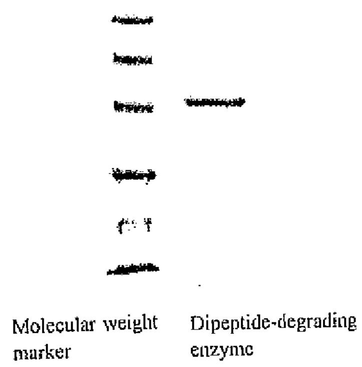

Fig.6

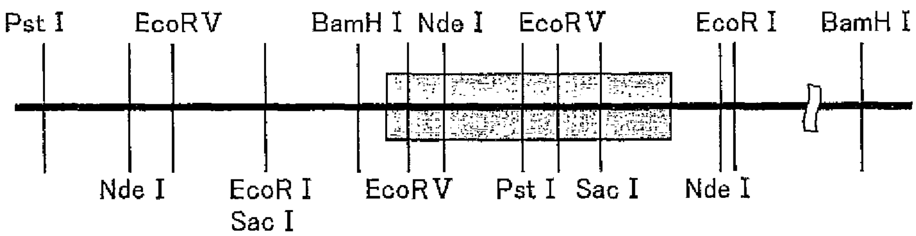
Pst I
EcoRV
BamH I
Nde I
EcoRV
EcoR I
BamH I
Nde I
EcoR I
Sac I
EcoRV
Pst I
Sac I
Nde I

Fig.7

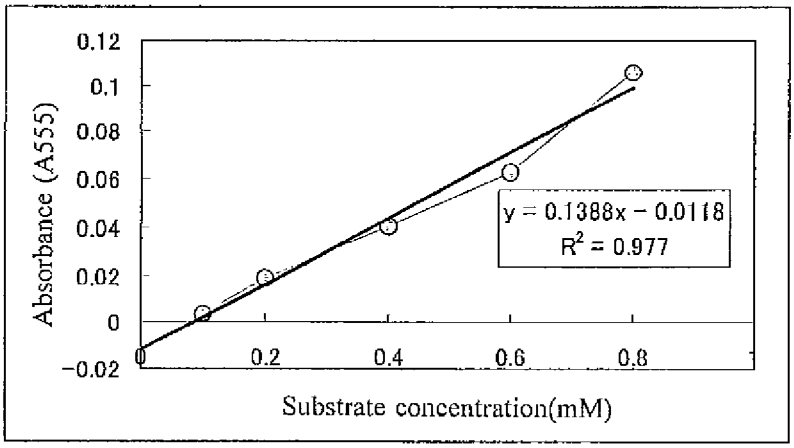
0.12
0.1
0.08
0.06
0.04
0.02
0
-0.02
0
0.2
0.4
0.6
0.8
Absorbance (A555)
Substrate concentration(mM)
y = 0.1388x - 0.0118
R2 = 0.977

Fig.8

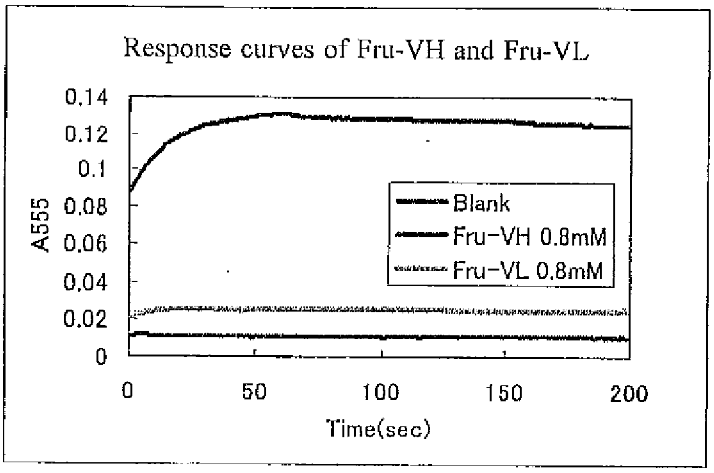
Response curves of Fru-VH and Fru-VL
0.14
0.12
0.1
0.08
0.06
0.04
0.02
0
A555
Blank
Fru-VH 0.8mM
Fru-VL 0.8mM
0
50
100
150
200
Time(sec)

**INTERNATIONAL SEARCH REPORT**

International application No.
PCT/JP2007/072093

A. CLASSIFICATION OF SUBJECT MATTER
*C12N15/00*(2006.01)i, *C12N1/21*(2006.01)i, *C12N9/48*(2006.01)i, *C12N15/09* (2006.01)i, *C12P13/04*(2006.01)i, *C12P19/02*(2006.01)i, *C12Q1/26*(2006.01)i, *C12Q1/37*(2006.01)i

According to International Patent Classification (IPC) or to both national classification and IPC

B. FIELDS SEARCHED

Minimum documentation searched (classification system followed by classification symbols)
C12N15/00-15/90, C12N9/00-9/99, C12P13/00-13/24, C12P19/00-21/06, C12Q1/26, C12Q1/37

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
UniProt/GeneSeq, GenBank/EMBL/DDBJ/GeneSeq, MEDLINE/CAPlus/BIOSIS/WPIDS(STN), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2000/050579 A1 (Kyoto Daiichi Kagaku Co., Ltd.),<br>31 August, 2000 (31.08.00),<br>& EP 1156105 A & AU 2576100 A<br>& CN 1340099 A | 1-3,16-24<br>26-27<br>4-15,25 |
| X<br>Y<br>A | WO 2000/061732 A1 (Arkray, Inc.),<br>19 October, 2000 (19.10.00),<br>& EP 1176191 A1 & US 2004/0247587 A1<br>& AU 3677100 A & CN 1355841 A | 1-3,16-24<br>26-27<br>4-15,25 |
| X<br>Y<br>A | JP 2006-94702 A (Daiichi Pure Chemicals Co., Ltd.),<br>13 April, 2006 (13.04.06),<br>& WO 2004/038035 A1 | 1-3,16-24<br>26-27<br>4-15,25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 December, 2007 (10.12.07) | 18 December, 2007 (18.12.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/072093

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2006-149230 A (Asahi Kasei Fama Kabushiki Kaisha),<br>15 June, 2006 (15.06.06),<br>(Family: none) | 26-27<br>1-25 |
| X<br>A | DUERRE, J.A. et.al., L-Amino Acid Oxidases of Proteus rettgeri.J. Bacteriol., 1975, vol.121, no.2, p.656-663 | 25<br>1-24,26-27 |
| X<br>A | JP 2005-110657 A (Kikkoman Corp.),<br>28 April, 2005 (28.04.05),<br>& WO 2005/028660 A1 & EP 1679378 A1<br>& US 2007/0037243 A1 | 25<br>1-24,26-27 |
| X<br>A | WO 2003/064683 A1 (Arkray, Inc.),<br>07 August, 2003 (07.08.03),<br>& EP 1477569 A1 & US 2005/0042709 A1<br>& CN 1625601 A | 25<br>1-24,26-27 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5033997 A
- JP 6065300 A
- JP 2195900 A
- JP 3155780 A
- JP 5192193 A
- JP 6046846 A
- JP 7289253 A
- JP 8154672 A
- JP 8336386 A
- JP 2005110657 A


### Non-patent literature cited in the description


- *Biotechnology Letters,* 2005, vol. 27, 963
- **F. Ausubel et al.** Current Protocols in Molecular Biology. John Wiley & Sons, Inc, 1994
- **R. Freshney.** Culture of Animal Cells; A Manual of Basic Technique. Wiley-Liss, 1987

SEQUENCE LISTING

<110> AMANO ENZYME INC.

<120> Novel dipeptidase, the method of producing the same, the use of the same to determine the glycated proteins

<130> P06A1

<160> 12

<170> PatentIn version 3.1

<210> 1
<211> 1374
<212> DNA
<213> Sphingopyxis chilensis No.0522

<400> 1
```
atgcgcagtc cgttcgcccc gctcgccctc gccgccgcgc tgatcgccgc ggcgcccgct      60

catgccaagc ctgccgatcc ggcggtggcg aagaagatcc tgatcgacag cgtcgcgatc     120

ccgaccgtcg aggggcgcgg caaggtgcct gaactcgcgg cctattatgc cggcgtgctc     180

aaggcggcgg gcttcaccga cgccgatatc gtcatcaccc cgattggcga gaccgcgagc     240

ttcgccgcaa cgctgaaagg caccagtgcc gaaaagccga tcctgctcct cggccatatg     300

gacgtcgtcg aggccgacgc gaaggactgg acgcgcgatc ccttcgtccc ggtcgaggag     360

aagggctata ttttcggccg cgggtcggag gacaacaagt tcgacgtcgc gatgatggtc     420

gcgacgatgg cccagctcaa aaaggacggt ttcaagccca ggcgttcgat catcctgctg     480

ctctcgggcg acgaggaaac cgcgatggtg acgacgcgcg cgcttgccgc ccaatataag     540

gacgccgaat ttgcgctgaa cggcgatggc ggcggcgggc tgatcggcga ggacggcaag     600

gcgaaatatt acgggctgca ggcgggcgaa aagacctacg ccgacttcac cctcgaagtc     660

accaaccccg gcggtcacag ctcgcgcccg tcggcgatca acgccatcgt ccagctttcg     720

accgcgctcg cgaagatcgg cgcctatcat ttcgcgcctc agcagaacga actgaccagg     780

gtcggcatgc cgatcgtcgc cgatcaggtc ggcggcgata tcggcgcggc gctcaaggct     840

tttgcggcca atccagccga cacggcggcg atcgcggcga tcaccgccga tcccgaatat     900

gtcgggcaga tcgggacgac ctgcgtgccg acgctcgtca agggcggcca tgccgaaaat     960

gcgttgccgc agcgcgccac cgcgaacatc aactgccgca tcttccccgg cgtcgagatc    1020

gaggcggtgc gcgccgagct cgaaaaggtc attgccgacc ccgcggtcgc ggtgaagacc    1080

gaccccgatg cgaccgcgag cgacgcctcg cccttgcgcc ccgacgtgat ggcggcggtg    1140

accaaggcgg tccacgcgcg cttccccggc ctgcccatca tcccgtcgat gagcgcgggc    1200

gcgaccgaca gcctccactt ccgcgccgtc ggcgtgccga gctatggcgt cgcggggctg    1260

ttttcgaagg cgagcgacag cttcgcccac ggcctcaacg aacgcgcgcc ggttgccgcg    1320

atccccggcg cgctcgcgca ttgggacagc ctgctgcgcg acctgtcgaa atag          1374
```

<210> 2
<211> 457

<212> PRT
<213> Sphingopyzis chilensis No.0522

<400> 2

```
Met Arg Ser Pro Phe Ala Pro Leu Ala Leu Ala Ala Ala Leu Ile Ala
1               5                   10                  15

Ala Ala Pro Ala His Ala Lys Pro Ala Asp Pro Ala Val Ala Lys Lys
            20                  25                  30

Ile Leu Ile Asp Ser Val Ala Ile Pro Thr Val Glu Gly Arg Gly Lys
        35                  40                  45

Val Pro Glu Leu Ala Ala Tyr Tyr Ala Gly Val Leu Lys Ala Ala Gly
    50                  55                  60

Phe Thr Asp Ala Asp Ile Val Ile Thr Pro Ile Gly Glu Thr Ala Ser
65                  70                  75                  80

Phe Ala Ala Thr Leu Lys Gly Thr Ser Ala Glu Lys Pro Ile Leu Leu
                85                  90                  95

Leu Gly His Met Asp Val Val Glu Ala Asp Ala Lys Asp Trp Thr Arg
            100                 105                 110

Asp Pro Phe Val Pro Val Glu Glu Lys Gly Tyr Ile Phe Gly Arg Gly
        115                 120                 125

Ser Glu Asp Asn Lys Phe Asp Val Ala Met Met Val Ala Thr Met Ala
    130                 135                 140

Gln Leu Lys Lys Asp Gly Phe Lys Pro Arg Arg Ser Ile Ile Leu Leu
145                 150                 155                 160

Leu Ser Gly Asp Glu Glu Thr Ala Met Val Thr Thr Arg Ala Leu Ala
                165                 170                 175

Ala Gln Tyr Lys Asp Ala Glu Phe Ala Leu Asn Gly Asp Gly Gly Gly
            180                 185                 190

Gly Leu Ile Gly Glu Asp Gly Lys Ala Lys Tyr Tyr Gly Leu Gln Ala
        195                 200                 205

Gly Glu Lys Thr Tyr Ala Asp Phe Thr Leu Glu Val Thr Asn Pro Gly
    210                 215                 220

Gly His Ser Ser Arg Pro Ser Ala Ile Asn Ala Ile Val Gln Leu Ser
225                 230                 235                 240

Thr Ala Leu Ala Lys Ile Gly Ala Tyr His Phe Ala Pro Gln Gln Asn
                245                 250                 255
```

```
Glu Leu Thr Arg Val Gly Met Pro Ile Val Ala Asp Gln Val Gly Gly
            260                 265                 270

Asp Ile Gly Ala Ala Leu Lys Ala Phe Ala Ala Asn Pro Ala Asp Thr
        275                 280                 285

Ala Ala Ile Ala Ala Ile Thr Ala Asp Pro Glu Tyr Val Gly Gln Ile
    290                 295                 300

Gly Thr Thr Cys Val Pro Thr Leu Val Lys Gly Gly His Ala Glu Asn
305                 310                 315                 320

Ala Leu Pro Gln Arg Ala Thr Ala Asn Ile Asn Cys Arg Ile Phe Pro
                325                 330                 335

Gly Val Glu Ile Glu Ala Val Arg Ala Glu Leu Glu Lys Val Ile Ala
            340                 345                 350

Asp Pro Ala Val Ala Val Lys Thr Asp Pro Asp Ala Thr Ala Ser Asp
        355                 360                 365

Ala Ser Pro Leu Arg Pro Asp Val Met Ala Ala Val Thr Lys Ala Val
    370                 375                 380

His Ala Arg Phe Pro Gly Leu Pro Ile Ile Pro Ser Met Ser Ala Gly
385                 390                 395                 400

Ala Thr Asp Ser Leu His Phe Arg Ala Val Gly Val Pro Ser Tyr Gly
                405                 410                 415

Val Ala Gly Leu Phe Ser Lys Ala Ser Asp Ser Phe Ala His Gly Leu
            420                 425                 430

Asn Glu Arg Ala Pro Val Ala Ala Ile Pro Gly Ala Leu Ala His Trp
        435                 440                 445

Asp Ser Leu Leu Arg Asp Leu Ser Lys
    450                 455
```

<210> 3
<211> 10
<212> PRT
<213> Sphingopyxis chilensis No.0522

<400> 3

```
Lys Pro Ala Asp Pro Ala Val Ala Lys Lys
1               5                   10
```

<210> 4
<211> 10
<212> PRT
<213> Sphingopyxis chilensis No.0522

<400> 4

Asp Ala Glu Phe Ala Leu Asn Gly Asp Gly
1               5                   10

<210> 5
<211> 9
<212> PRT
<213> Sphingopyxis chilensis No.0522

<400> 5

Tyr Tyr Gly Leu Gln Ala Gly Glu Lys
1               5

<210> 6
<211> 17
<212> DNA
<213> Artificial Sequence

<220>
<223> Description of Artificial Sequence: Synthetic DNA(Primer)

<220>
<221> misc_feature
<222> (3)..(3)
<223> n stands for any base (a or g or c or t)

<220>
<221> misc_feature
<222> (9)..(9)
<223> n stands for any base (a or g or c or t)

<220>
<221> misc_feature
<222> (12)..(12)
<223> n stands for any base (a or g or c or t)

<220>
<221> misc_feature
<222> (15)..(15)
<223> n stands for any base (a or g or c or t)

<400> 6
gcngayccng cngtngc 17

<210> 7
<211> 23
<212> DNA
<213> Artificial Sequence

<220>
<223> Description of Artificial Sequence: Synthetic DNA(Primer)

<220>
<221> misc_feature
<222> (6)..(6)
<223> n stands for any base (a or g or c or t)

<220>
<221> misc_feature
<222> (9)..(9)
<223> n stands for any base (a or g or c or t)

<220>
<221> misc_feature
<222> (18)..(18)
<223> n stands for any base (a or g or c or t)

<400> 7
ccrttnarng craaytcngc rtc 23

<210> 8
<211> 479
<212> DNA
<213> Sphingopyxis chilensis No.0522

<400> 8
gacccggcgg tggcgaagaa gatcctgatc gacagcgtcg cgatcccgac cgtcgagggg 60

cgcggcaagg tgcctgaact cgcggcctat tatgccggcg tgctcaaggc ggcgggcttc 120

accgacgccg atatcgtcat caccccgatt ggcgagaccg cgagcttcgc cgcaacgctg 180

aaaggcacca gtgccgaaaa gccgatcctg ctcctcggcc atatggacgt cgtcgaggcc 240

gacgcgaagg actggacgcg cgatcccttc gtcccggtcg aggagaaggg ctatattttc 300

ggccgcgggt cggaggacaa caagttcgac gtcgcgatga tggtcgcgac gatggcccag 360

ctcaaaaagg acggtttcaa gcccaggcgt tcgatcatcc tgctgctctc gggcgacgag 420

gaaaccgcga tggtgacgac gcgcgcgctt gccgcccaat ataaggacgc cgagttcgc 479

<210> 9
<211> 27
<212> DNA
<213> Artificial Sequence

<220>
<223> Description of Artificial Sequence: Synthetic DNA(Primer)

<400> 9
gacccggcgg tggcgaagaa gatcctg 27

<210> 10
<211> 29
<212> DNA
<213> Artificial Sequence

<220>
<223> Description of Artificial Sequence: Synthetic DNA(Primer)

<400> 10
gcgaactcgg cgtccttata ttgggcggc 29

<210> 11
<211> 29
<212> DNA
<213> Artificial Sequence

```
<220>
<223>  Description of Artificial Sequence: Synthetic DNA(Primer)

<400>  11
gatcagtcat gagaagtccg ttcgccccg                                   29


<210>  12
<211>  33
<212>  DNA
<213>  Artificial Sequence

<220>
<223>  Description of Artificial Sequence: Synthetic DNA(Primer)

<400>  12
ctgatcgtcg acctatttcg acaggtcgcg cag                              33
```